# EUROPEAN PATENT APPLICATION

(11) **EP 4 742 251 A1**
(43) Date of publication of application: **13.05.2026**
(21) Application number: 25745250.8
(22) Date of filing: 24.01.2025
(51) Int. Cl.: G16C 20/40, G16C 20/70, G16C 20/80, G06N 20/00

(54) **DEVICE AND METHOD FOR MEASURING RELIABILITY OF MOLECULAR STRUCTURE PREDICTION MODEL**

(30) Priority: 24.01.2024 KR 20240011080
(71) Applicant: LG Management Development Institute Co., Ltd., Seoul 07336 (KR)
(72) Inventor: KIM, Jiye, Goyang-si Gyeonggi-do 10372 (KR); JO, Yeonsik, Seoul 07791 (KR); LEE, Soonyoung, Seoul 06299 (KR)
(74) Representative: BCKIP Part mbB
(86) International application number: PCT/KR2025/001506
(87) International publication number: WO 2025/159584

(57) **Abstract**

A system, a computer program, a device, and a method for measuring confidence of a molecular structure prediction model. The method includes obtaining a first molecular structure image, obtaining a first molecular structure graph using the molecular structure prediction model, performing image rendering on the first molecular structure image based on the first molecular structure graph, and determining confidence of the first molecular structure graph based on the image rendering result and the first molecular structure graph.

## Description

### [Technical Field]

Embodiments of the invention relate generally to a device and a method for measuring the confidence of a molecular structure prediction model, and more specifically, the invention provides convenience by providing the confidence in the result when a molecular structure prediction model provides a predicted molecular structure.

### [Background Art]

A structural formula is a graphical representation of a chemical structure or a molecular structure and may show how atoms are arranged in a three-dimensional space. The structural formula may clearly or implicitly indicate chemical bonds of a molecule. In particular, unlike a molecular formula that has a limited number of symbols and may only provide limited descriptions, the structural formula may provide geometric information of the molecular structure. For example, isomers having the same molecular formula but different atomic structures or arrangements may be represented.

In various documents, papers, patents, etc., the structural formulas are often provided in the form of images. However, unlike text, images are difficult to search, making it difficult to find documents that include the corresponding structural formula. Accordingly, various methods for searching images such as the structural formula are being developed. Models for extracting the structural formulas by analyzing images are mainly used to create academic databases, and when incorrect data is included in such academic databases due to erroneous predictions, it becomes a critical drawback for research. Accordingly, there is a need for a method that provides confidence information about predicted structural formulas to determine which predicted structural formulas should be regarded as reliable information and stored in a database.

### [Disclosure]

### [Technical Problem]

Embodiments of the invention provide a method and a device in which a model that predicts a molecular structure using an image provides a confidence score together when predicting the molecular structure.

### [Technical Solution]

One embodiment of the present disclosure may provide a device and a method for measuring the confidence of a molecular structure prediction model.

According to one or more embodiments of the invention, a system for measuring the confidence of the molecular structure prediction model is provided. The system includes a memory storing one or more instructions, and at least one processor configured to execute the one or more instructions stored in the memory. The at least one processor, by executing the one or more instructions, may obtain a first molecular structure image, obtain a first molecular structure graph determined using the molecular structure prediction model, perform image rendering on the first molecular structure image based on the first molecular structure graph, and determine the confidence of the first molecular structure graph based on the image rendering result and the first molecular structure graph.

In one embodiment, the at least one processor may identify at least one of a first component and a second component based on the first molecular structure graph, identify a first portion corresponding to the first component in the first molecular structure image, identify a second portion corresponding to the second component in the first molecular structure image, and perform the image rendering by distinguishing the first portion and the second portion using different markings. Each of the first component and the second component may include one of a first atom, a second atom, a first bond, and a second bond.

In one embodiment, the molecular structure prediction model may include a first learning model, wherein the first learning model may be trained to extract a chemical table file graph with a molecular structural formula image as input.

In one embodiment, the at least one processor may output the confidence using a second learning model with the image rendering result and the first molecular structure graph as input.

In one embodiment, the second learning model may include an image backbone model configured to extract a feature of the image rendering result, a graph backbone model configured to extract a feature of the first molecular structure graph, a feature concatenation unit configured to concatenate the feature of the image rendering result and the feature of the first molecular structure graph, and a linear layer model configured to determine the confidence with an output of the feature concatenation unit as input.

In one embodiment, the second learning model may be trained to output a first value when the image rendering result matches the first molecular structure graph and to output a second value when the image rendering result does not match the first molecular structure graph.

In one embodiment, the graph of the molecular structure with the confidence equal to or greater than a predetermined level may be stored in a database.

According to yet another embodiment of the invention, a method for measuring confidence of a molecular structure prediction model, performed by at least one processor, is provided. The method may include obtaining a first molecular structure image, obtaining a first molecular structure graph using the molecular structure prediction model, performing image rendering on the first molecular structure image based on the first molecular structure graph, and determining the confidence of the first molecular structure graph based on the image rendering result and the first molecular structure graph.

In one embodiment, the performing of the image rendering on the first molecular structure image may include identifying at least one of a first component and a second component based on the first molecular structure graph, identifying a first portion corresponding to the first component in the first molecular structure image, identifying a second portion corresponding to the second component in the first molecular structure image, and performing the image rendering by distinguishing the first portion and the second portion using different markings. Each of the first component and the second component may include one of a first atom, a second atom, a first bond, and a second bond.

In one embodiment, the molecular structure prediction model may include a first learning model trained to extract a chemical table file graph with a molecular structural formula image as input.

In one embodiment, the determining of the confidence of the first molecular structure graph may include outputting the confidence of the first molecular structure graph using a second learning model with the image rendering result and the first molecular structure graph as input.

In one embodiment, the second learning model may include an image backbone model configured to extract a feature of the image rendering result, a graph backbone model configured to extract a feature of the first molecular structure graph, a feature concatenation unit configured to concatenate the feature of the image rendering result and the feature of the first molecular structure graph, and a linear layer model configured to determine the confidence with an output of the feature concatenation unit as input.

In one embodiment, the second learning model may be trained to output a first value when the image rendering result matches the first molecular structure graph and to output a second value when the image rendering result does not match the first molecular structure graph.

In one embodiment, the graph of the molecular structure with the confidence equal to or greater than a predetermined value may be stored in a database.

A computer program may be installed in an information processing device and stored in a non-transitory recording medium to execute the method according an embodiment of the invention.

A non-transitory computer-readable recording medium may be provided in which a program for executing the method according to an embodiment of the invention on a computer is recorded.

A non-transitory computer-readable recording medium may be provided in which a database used in an embodiment of the invention is recorded.

### [Advantageous Effects]

According to an embodiment of the present disclosure, accuracy information about the result of the predictive model may be provided by providing the reliability of the result of the predictive model together, and furthermore, may help the user determine what information to store in the database.

### [Description of Drawings]

FIG. 1 is a diagram showing a method for extracting a molecular structural formula from an image using a first learning model according to one embodiment of the present disclosure.
FIG. 2 is a flowchart showing a method for providing confidence of a result value of the first learning model using a second learning model according to one embodiment of the present disclosure.
FIG. 3 is a flowchart showing a method for measuring the confidence of a molecular structure prediction model according to one embodiment of the present disclosure.
FIG. 4A is a diagram showing a method for measuring the confidence when the molecular structure prediction model makes an incorrect prediction according to one embodiment of the present disclosure.
FIG. 4B is a diagram showing a method for measuring the confidence when the molecular structure prediction model makes a correct prediction according to one embodiment of the present disclosure.
FIG. 5 is an example diagram of the second learning model according to one embodiment of the present disclosure.
FIG. 6 is a block diagram of a device for measuring the confidence of the molecular structure prediction model according to one embodiment of the present disclosure.

### [Mode for Invention]

In order to clarify the technical idea of the present disclosure, embodiments of the present disclosure will be described in detail with reference to the accompanying drawings. In describing the present disclosure, when it is determined that the detailed description of a related known function or component may unnecessarily obscure the gist of the present disclosure, the detailed description thereof will be omitted. In the drawings, components having substantially the same function or configuration are denoted as the same reference numeral and symbol as much as possible even when they are shown in different drawings. For convenience of explanation, a device and a method will be described together when necessary. Each operation of the present disclosure does not necessarily need to be performed in the order described, and may be performed in parallel, selectively, or individually.

The terms used in the embodiments of the present disclosure were selected as general terms widely used at present as possible while considering the function of the present disclosure, but these terms may vary depending on the intention of those skilled in the art, legal precedents, the emergence of new technologies, etc. In addition, in specific cases, there are terms arbitrarily selected by the applicant, and in this case, the meanings thereof will be described in detail in the description of the corresponding embodiment. Therefore, the terms used in the present specification should be defined based on the meanings of the terms and the overall contents of the present disclosure rather than just the names of the terms.

Throughout the present disclosure, singular expressions may include plural expressions unless the context explicitly states otherwise. It should be understood that terms such as "comprise" or "have" are intended to specify the presence of a feature, number, step, operation, component, part, or a combination thereof, but do not preemptively preclude the possibility of the presence or addition of one or more other features, numbers, steps, operations, components, parts, or combinations thereof. That is, throughout the present disclosure, when a certain portion is described as "including," a certain component, it means further including another component rather than precluding another component unless especially stated otherwise.

Expressions such as "at least one" modify the entire list of components, and do not individually modify components of the list. For example, "at least one of A, B, and C" or "at least one of A, B, or C" refers to only A, only B, only C, both A and B, both B and C, both A and C, all of A, B, and C, or a combination thereof.

In addition, terms such as "...unit," "...module," etc. described in the present disclosure mean a unit that process at least one function or operation, which may be implemented as hardware or software, or a combination of hardware and software.

Throughout the present disclosure, when a certain portion is described as being "connected" to another portion, it includes not only a case where the certain portion is "directly connected" to another portion but also a case where the certain portion is "electrically connected" to another portion with another element interposed therebetween. In addition, when a certain portion is described as "including" a certain component, it means further including another component rather than precluding another component unless specifically stated otherwise.

The expression "configured to (or set to)" as used throughout the present disclosure may, depending on the contexts, be used interchangeably with, for example, "suitable for," "having the capacity to," "designed to," "adapted to," "made to," or "capable of." The term "configured to (or set to)" does not necessarily mean only "specifically designed to" in hardware. Instead, in certain contexts, the expression "a system configured to" may mean that the system is "capable of" something along with other devices or parts. For example, the phrase "a processor configured to (or set to) perform A, B, and C" may mean a dedicated processor (e.g., an embedded processor) for performing corresponding operations, or a generic-purpose processor (e.g., a CPU or application processor) that can perform corresponding operations by executing one or more software programs stored in a memory.

The functions related to artificial intelligence according to the present disclosure are operated through a processor and a memory. The processor may include one or a plurality of processors. In this case, the one or plurality of processors may be a general-purpose processor such as a CPU, an AP, or a digital signal processor (DSP), a graphics-dedicated processor such as a graphics processing unit (GPU) or a vision processing unit (VPU), or an artificial intelligence-dedicated processor such as a neural processing unit (NPU). The one or plurality of processors may control input data to be processed according to a predefined operation rule or an artificial intelligence model that are stored in the memory. Alternatively, when the one or plurality of processors are artificial intelligence-dedicated processors, the artificial intelligence-dedicated processor may be designed with a hardware structure specialized for processing a specific artificial intelligence model.

It is characterized in that the predefined operation rule or the artificial intelligence model are generated through training. Here, being generated through training means that a basic artificial intelligence model is trained using a plurality of training data by a learning algorithm, thereby generating the predefined operation rule or the artificial intelligence model that are set to perform a desired characteristic (or objective). Such training may be performed on a device itself on which the artificial intelligence according to the present disclosure is performed, or may be performed through a separate server and/or system. Examples of the learning algorithm may include supervised learning, unsupervised learning, semi-supervised learning, or reinforcement learning, but are not limited thereto.

Throughout the present disclosure, the device may include a server, a smartphone, a tablet PC, a PC, a TV, a smart TV, a mobile phone, a personal digital assistant (PDA), a speaker, a laptop, a media player, a micro server, an e-book object recognition device, a digital broadcasting object recognition device, a kiosk, an MP3 player, a digital camera, a robot vacuum cleaner, home appliances, other mobile or non-mobile computing devices, or a watch, glasses, a hairband, or a ring that has a communication function and a data processing function, but is not limited thereto. In one embodiment, the device may execute a web-based or module-based application related to a system. For example, the device for measuring confidence of a molecular structure prediction model may refer to a server, and a web-based application related to a system for measuring the confidence of the molecular structure prediction model may be executed on the server. That is, the server may provide a web service or software that measures the confidence of the molecular structure prediction model.

One embodiment of the present disclosure is directed to predicting a molecular structural formula from a molecular structure image and providing confidence for the predicted molecular structural formula. However, the present disclosure is not limited to the embodiment of the molecular structure, and it goes without saying that it may be applied to various technical fields that extract image information from images rather than text.

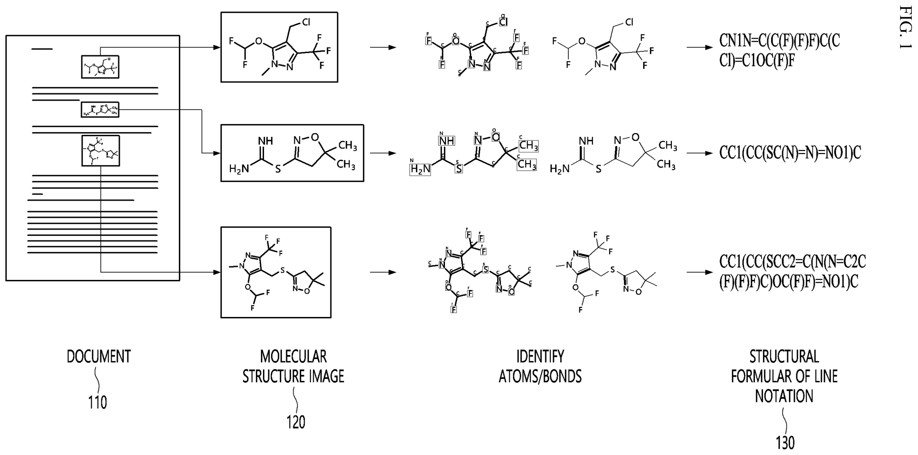

FIG. 1 is a diagram showing a method for extracting a molecular structural formula from an image using a first learning model according to one embodiment of the present disclosure.

Referring to FIG. 1, a device including a molecular structure prediction model may identify a molecular structure image 120 from a document 110 and analyze the image 120 to extract the molecular structure corresponding to the corresponding image. In one embodiment, one device may include the molecular structure prediction model and a confidence measurement model, or the molecular structure prediction model and the confidence measurement model may each be included in different devices so that each device calculates an output value using the corresponding model.

In one embodiment, the device including the molecular structure prediction model may extract the molecular structure image 120 from the document 110. For example, the device including the molecular structure prediction model may extract the molecular structure image 120 from the document 110 using a panoptic segmentation technique. In addition, the device including the molecular structure prediction model may identify atoms and bonds between the atoms, which are included in the molecular structure image 120, based on the molecular structure image 120. In one embodiment, the device including the molecular structure prediction model may analyze types and positions of the atoms, types and positions of the bonds, and the like, which are included in the molecular structure image 120. For example, vertices implicitly representing carbon, the atoms, superatoms, etc. and their positions, the types of the bonds therebetween (e.g., single/double/triple bonds, up/down bonds, and the like) may be identified. In analyzing the types of the atoms or the superatoms, optical character recognition (OCR) technology may be used. In one embodiment, the device including the molecular structure prediction model may determine a structural formula 130 of line notation corresponding to the molecular structure image 120, based on the types and the positions of the atoms, the types and the positions of the bonds, and the like, which are identified. The structural formula 130 of the line notation may include simplified molecular input line entry system (SMILES) notation, international chemical identifier (InChI), Wiswesser line notation (WLN), representation of organic structure descriptions arranged linearly (ROSDAL), SYBYL line notation (SLN), and the like, but is not limited thereto. Furthermore, the device including the molecular structure prediction model may determine a graph corresponding to the structural formula 130 of the line notation. The graph corresponding to the structural formula 130 of the line notation may include a chemical table file (CT file) graph, and may include, for example, a molfile graph.

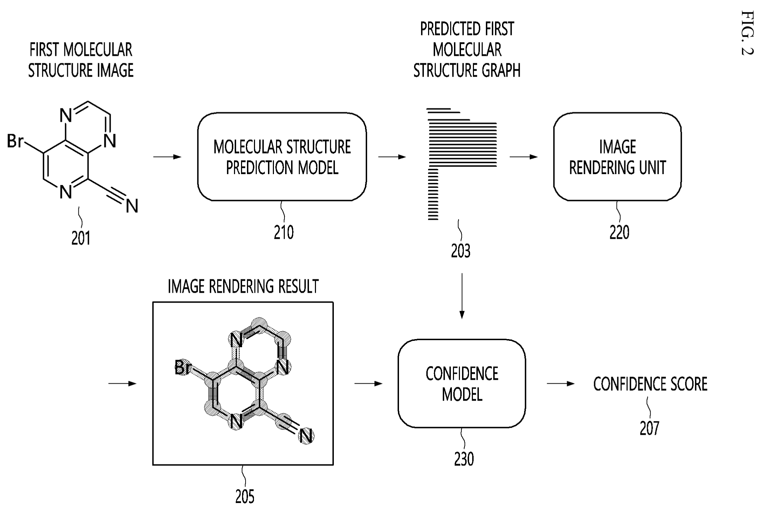

Conventionally, in providing the structural formula 130 of the line notation or the graph that is predicted in this way, a method has been used in which a chemical structural formula is generated from the predicted result and the confidence is measured when the generated structural formula is actually bondable, in order to determine whether the predicted result is reliable. However, since the method does not consider the actual image, there is a high probability that incorrect data will be accumulated by outputting a high confidence score when the structural formula is determined to be actually bondable even though the prediction is incorrect. Accordingly, a method for providing a more accurate confidence score of the prediction model is required, and this will be described in more detail below with reference to FIGS. 2 to 5.

FIG. 2 is a flowchart showing a method for providing confidence of a result value of the first learning model using a second learning model according to one embodiment of the present disclosure.

Referring to FIG. 2, a predicted first molecular structure graph 203 corresponding to a first molecular structure image 201 may be obtained using a molecular structure prediction model 210. The method for obtaining the predicted first molecular structure graph 203 may be implemented using the above-described methods with reference to FIG. 1.

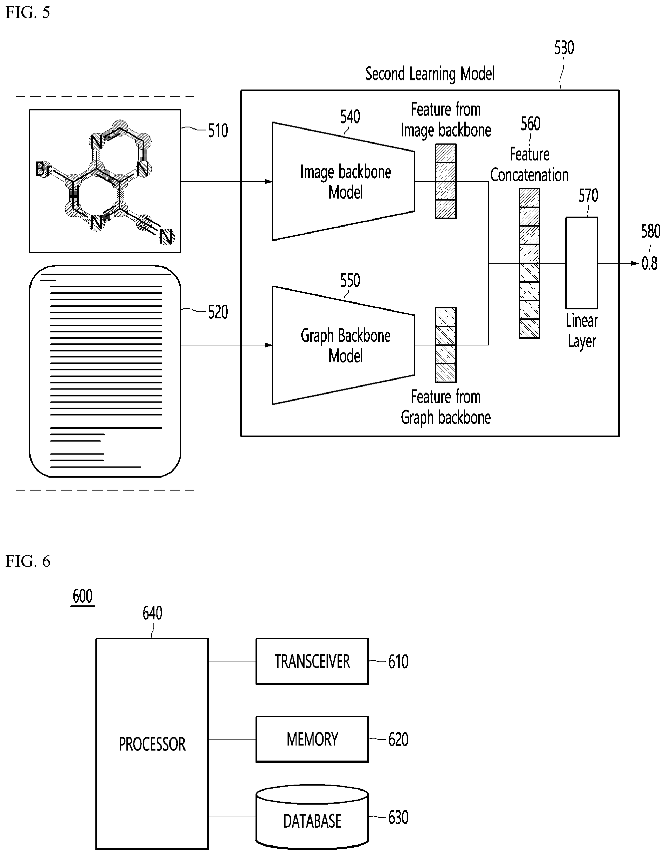

In one embodiment, image rendering may be performed by an image rendering unit 220 based on the first molecular structure graph 203 predicted by the molecular structure prediction model 210. The image rendering may be performed by identifying a plurality of components based on the first molecular structure graph 203, identifying a portion corresponding to each of the plurality of components, and marking the portions on the first molecular structure image 201, which is an original image, with different markings to distinguish between different components. Furthermore, in order to identify the portion corresponding to each of the plurality of components in the first molecular structure image 201, a position of each of the plurality of components may also be identified. The plurality of components may include a first atom, a second atom, a third atom, a first bond, a second bond, a third bond, and the like. For example, referring to the example in FIG. 2, a device for measuring the confidence of the molecular structure prediction model 600 (see FIG. 6) may identify bromine (Br), four nitrogens (N), eight vertices representing carbon (C), seven single bonds, five double bonds, and one triple bond based on the first molecular structure graph 203, and may mark elements with circles and the bonds with line segments, and may use different colors for different elements or different bonds. Accordingly, an image rendering result 205 of FIG. 2 may be obtained by the image rendering unit 220.

In one embodiment, an image rendering result 205 and the predicted first molecular structure graph 203 are input to a confidence model 230, and a confidence score 207 may be obtained based on the degree of similarity between the two inputs. The more similar the input information of the two input values is, the higher the confidence score 207 may be obtained, and the less similar the input information is, the lower the confidence score 207 may be obtained.

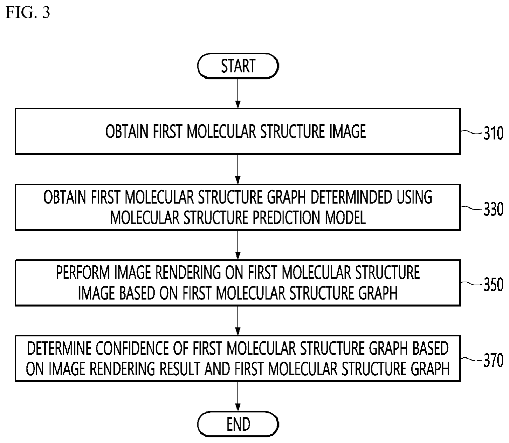

FIG. 3 is a flowchart showing a method for measuring the confidence of the molecular structure prediction model according to one embodiment of the present disclosure.

Referring to FIG. 3, in step 310, the device for measuring the confidence of the molecular structure prediction model 600 may obtain the first molecular structure image 201. In one embodiment, the device for measuring the confidence of the molecular structure prediction model 600 may obtain the first molecular structure image 201 by extracting the first molecular structure image 201 directly from a document, or obtain the first molecular structure image 201 extracted from another device. That is, the device for measuring the confidence of the molecular structure prediction model 600 or the external device may extract the first molecular structure image 201 using an artificial intelligence (AI) algorithm or a predefined operation rule.

In step 330, the device for measuring the confidence of the molecular structure prediction model 600 may obtain the first molecular structure graph 203 determined using the molecular structure prediction model 210. That is, the first molecular structure graph 203 may be a graph corresponding to the first molecular structure image 201 predicted by the molecular structure prediction model 210. In one embodiment, the molecular structure prediction model 210 may include a first learning model trained to output a chemical table file (CT file) graph with an image of a molecular structural formula as input. The CT file graph may include information about each atom in a molecule, x-y-z coordinate information of the atom, bonding information between the atoms, and the like. As in step 310, the device for measuring the confidence of the molecular structure prediction model 600 may directly obtain the first molecular structure graph 203 determined using the molecular structure prediction model 210, or may obtain the first molecular structure graph 203 determined using the molecular structure prediction model 210 by receiving the graph from an external device.

In step 350, the device for measuring the confidence of the molecular structure prediction model 600 may perform image rendering on the first molecular structure image 201 based on the first molecular structure graph 203. In one embodiment, the device for measuring the confidence of the molecular structure prediction model 600 may identify a plurality of components based on the first molecular structure graph 203, identify a portion corresponding to each of the plurality of components in the first molecular structure image 201, and perform image rendering by distinguishing different components with different markings. Here, the plurality of components may include the atoms, bonds between the atoms, and the like. Distinguishing different components with different markings may include distinguishing with different colors or different shapes, but is not limited thereto and may include various marking forms that distinguish one from another.

For example, when a first component is carbon and a second component is nitrogen, the device for measuring the confidence of the molecular structure prediction model 600 may identify the carbon, which is the first component, and its position coordinate, and the nitrogen, which is the second component, and its position coordinate, in the first molecular structure graph 203, identify a first portion corresponding to the first component and a second portion corresponding to the second component in the first molecular structure image 201 based on each position coordinate, and perform image rendering by distinguishing the first portion and the second portion with different markings, such as marking the first portion in yellow and the second portion in red.

As another example, when the first component is carbon, the second component is nitrogen, a third component is a single bond, and a fourth component is a double bond, the device for measuring the confidence of the molecular structure prediction model 600 may identify the carbon, which is the first component, and its position, identify the nitrogen, which is the second component, and its position, identify a position of the single bond, which is the third component, and identify a position of the double bond, which is the fourth component, in the first molecular structure graph 203. In addition, the device for measuring the confidence of the molecular structure prediction model 600 may perform image rendering by identifying the first portion corresponding to the first component, the second portion corresponding to the second component, a third portion corresponding to the third component, and a fourth portion corresponding to the fourth component in the first molecular structure image 201 based on the position of each component, by marking the first portion and the second portion, which correspond to the elements, in the form of circles with different colors to indicate different elements, and by marking the third portion and the fourth portion, which correspond to the bonds, in the form of bars with different colors to indicate different types of bonds.

In step 370, the device for measuring the confidence of the molecular structure prediction model 600 may determine the confidence of the first molecular structure graph 203 based on the image rendering result 205 and the first molecular structure graph 203. In one embodiment, the confidence of the first molecular structure graph 203 may be determined using a second learning model. The second learning model may be a learning model that outputs a confidence value (e.g., a confidence score 207) with the image rendering result 205 and the first molecular structure graph 203 as input. In one embodiment, the learning model may include an image backbone network model that extracts features of the image rendering result, a graph backbone network model that extracts features of the first molecular structure graph, a feature concatenation unit that concatenates the extracted features of the image rendering result and the extracted features of the first molecular structure graph, a network model that extracts confidence with the output of the feature concatenation unit as input, or the like. This will be described in more detail below with reference to FIG 5.

In one embodiment, the second learning model may be trained to output a first value when the image rendering result 205 matches the first molecular structure graph 203, and to output a second value when the image rendering result 205 does not match the first molecular structure graph 203. For example, in the learning phase, the second learning model may be trained to output 1 when the image rendering result 205 matches the first molecular structure graph 203 and to output 0 when the image rendering result 205 does not match the first molecular structure graph 203, so that, after training is completed, when the second learning model is actually used, a value close to 1 may be extracted when the prediction result of the molecular structure prediction model 210 is accurate, and a value close to 0 may be extracted when the prediction contains errors. The device for measuring the confidence of the molecular structure prediction model 600 may determine the confidence of the first molecular structure graph 203 based on the value extracted by the second learning model. For example, the confidence may be determined as a confidence value (or score 207).

According to one embodiment, since the device for measuring the confidence of the molecular structure prediction model 600 provides the confidence together with the predicted graph of the first molecular structure, thereby providing the accuracy of the predicted graph of the first molecular structure together, the device for measuring the confidence of the molecular structure prediction model 600 may help determine whether to utilize the predicted graph. Accordingly, a user or the user's device may determine to store the graph in a database only when the confidence is equal to or greater than a predetermined level (or value), thereby enabling accurate data to be utilized for research, and the like.

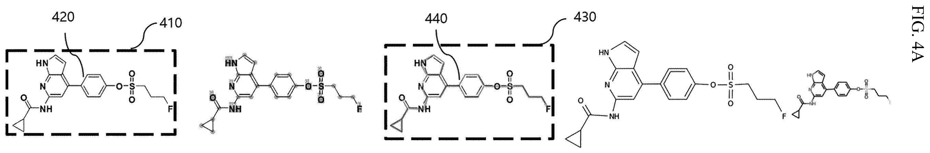

FIG. 4A is a diagram showing a method for measuring the confidence when the molecular structure prediction model makes an incorrect prediction according to one embodiment of the present disclosure.

Referring to FIG. 4A, a first molecular structure may be predicted by the molecular structure prediction model 210 based on a first molecular structure image 410. For example, the first molecular structure may be a structure formed by the coexistence of single bond and the double bond. However, unlike humans, a machine may not be expected to achieve 100% accuracy in predicting the first molecular structure based on the first molecular structure image 410. Accordingly, a process is required to confirm whether the first molecular structure predicted based on the first molecular structure image 410 has been correctly predicted.

In one embodiment, the device for measuring the confidence of the molecular structure prediction model 600 may obtain the first molecular structure image 410 and the first molecular structure graph 203 predicted by the first learning model. In addition, the device for measuring the confidence of the molecular structure prediction model 600 may identify the type of the element, the position of the element, the type of the bond, the position of the bond, and the like based on the predicted first molecular structure graph 203, and may perform rendering on the first molecular structure image 410.

For example, when a first bond 420 is identified as a double bond in the first molecular structure graph 203, the device for measuring the confidence of the molecular structure prediction model 600 may generate an image rendering result 430 by marking the bonds identified as double bonds including the first bond 440 and the bonds identified as single bonds with different colors on the first molecular structure image 410. That is, the first bond 420 is actually a single bond, but in the image rendering result 205, the first bond 420 may appear to have a double bond.

In one embodiment, the device for measuring the confidence of the molecular structure prediction model 600 may determine the confidence of the first molecular structure graph 203 based on the image rendering result 430. For example, the device for measuring the confidence of the molecular structure prediction model 600 may identify element portions and bond portions using segmentation of the first molecular structure image 410, and identify whether the elements are the same or different from each other, or whether the bonds are the same or different from each other. When the segmentation result of the first molecular structure image 410 is similar to the image rendering result 430, the device for measuring the confidence of the molecular structure prediction model 600 may determine that the confidence is high, and when the segmentation result is not similar to the image rendering result 430, the device may determine that the confidence is low. In the example of FIG. 4A, since it is determined in the image rendering result 430 that the first bond 440 has the double bond, but it is determined in the first molecular structure image 410 that the first bond 420 has the single bond, the confidence of the first molecular structure graph 203 may be determined to be low. Alternatively, the confidence score of the first molecular structure graph 203 may be determined to be low.

Alternatively, in one embodiment, the device for measuring the confidence of the molecular structure prediction model 600 may determine the confidence of the first molecular structure graph 203 using both the image rendering result 430 and the predicted first molecular structure graph 203. According to one embodiment, the molecular structure graph may also be used to determine the confidence, thereby increasing the accuracy of the determined confidence. This will be described in more detail below with reference to FIG 5.

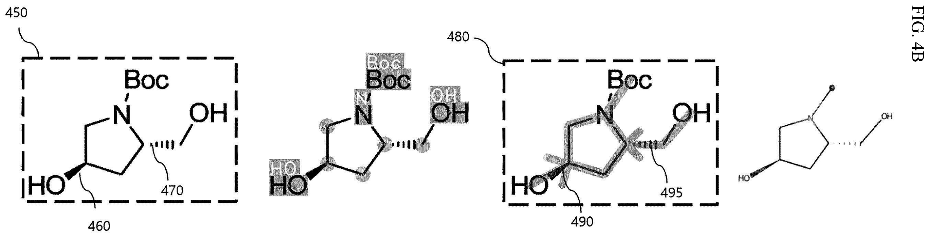

FIG. 4B is a diagram showing a method for measuring the confidence when the molecular structure prediction model 210 makes a correct prediction according to one embodiment of the present disclosure.

Referring to FIG. 4B, a second molecular structure may be predicted by the molecular structure prediction model 210 based on a second molecular structure image 450. However, as described above with respect to FIG. 4A, unlike humans, a machine may not be expected to achieve 100% accuracy in predicting the second molecular structure based on the second molecular structure image 450. Accordingly, a process is required to confirm whether the second molecular structure predicted based on the second molecular structure image 450 has been correctly predicted.

In one embodiment, the device for measuring the confidence of the molecular structure prediction model 600 may obtain the second molecular structure image 450 and the second molecular structure graph (not shown) predicted by the first learning model. In addition, the device for measuring the confidence of the molecular structure prediction model 600 may identify the type of the element, the position of the element, the type of the bond, the position of the bond, and the like based on the predicted second molecular structure graph, and may perform rendering on the second molecular structure image 450.

For example, when the second molecular structure graph shows that bonds other than a second bond 460 and a third bond 470 are single bonds, and the second bond 460 is a bond protruding out of the plane, and the third bond 470 is a bond recessed behind the plane, the device for measuring the confidence of the molecular structure prediction model 600 may generate an image rendering result 480 by marking a second bond 490, a third bond 495, and the other bonds with different colors on the second molecular structure image 450. Furthermore, the device for measuring the confidence of the molecular structure prediction model 600 may generate the image rendering result 480 to represent the bond positions of the second bond and the third bond.

In one embodiment, the device for measuring the confidence of the molecular structure prediction model 600 may determine the confidence of the second molecular structure graph based on the image rendering result 480. For example, the device for measuring the confidence of the molecular structure prediction model 600 may identify element portions and bond portions using segmentation of the second molecular structure image 450, and identify whether the elements are the same or different from each other, or whether the bonds are the same or different from each other. When the segmentation result of the second molecular structure image 450 is similar to the image rendering result 480, the device for measuring the confidence of the molecular structure prediction model 600 may determine that the confidence is high, and when the segmentation result is not similar to the image rendering result 430, the device may determine that the confidence is low. In the example of FIG. 4B, since the image rendering result 480 is similar to the second molecular structure image 450, the confidence of the second molecular structure graph may be determined to be high. Alternatively, the confidence score of the second molecular structure graph may be determined to be high.

Alternatively, in one embodiment, the device for measuring the confidence of the molecular structure prediction model 600 may determine the confidence of the second molecular structure graph based on the image rendering result and the predicted second molecular structure graph. This will be described in more detail below with reference to FIG. 5.

FIG. 5 is an example diagram of the second learning model according to one embodiment of the invention.

Referring to FIG. 5, a second learning model 530 for measuring the confidence may include an image backbone model 540 that extracts features of an image rendering result 510, a graph backbone model 550 that extracts features of a molecular structure graph 520, a feature concatenation unit 560 that concatenates the extracted features of the image rendering result 510 and the extracted features of the molecular structure graph 520, a network model 570 that determines the confidence with the output of the feature concatenation unit 560 as input, and the like. The network model 570 that determines the confidence may be a model that classifies the concatenated features through a linear layer.

In one embodiment, the second learning model 530 may be an artificial intelligence model that extracts a confidence value 580 with the image rendering result 510 and the molecular structure graph 520 as input. In this case, the image rendering result 510 may be input to the image backbone model 540, and the molecular structure graph 520 may be input to the graph backbone model 550, thus serving as input to different network models. The image backbone model 540 may extract the features of the image rendering result 510, and the graph backbone model 550 may extract the features of the molecular structure graph 520. The feature concatenation unit 560 may concatenate the features extracted from each backbone model, and the network model 570 that determines the confidence may determine a confidence value based on the extracted features. For example, as the confidence increases, a higher value may be output, and as the confidence decreases, a lower value may be output.

FIG. 6 is a block diagram of the device for measuring the confidence of the molecular structure prediction model 600 according to one embodiment of the present disclosure.

Referring to FIG. 6, the device for measuring the confidence of the molecular structure prediction model 600 may include a transceiver 610, a memory 620, a database 630, and a processor 640. However, not all of the components shown in FIG. 6 are essential components for the device for measuring the confidence of the molecular structure prediction model 600. The device for measuring the confidence of the molecular structure prediction model 600 may be implemented by more or fewer components than those shown in FIG. 6. In addition, the transceiver 610, the memory 620, and the processor 640 may be implemented in the form of a single chip.

In one embodiment, the transceiver 610 may communicate with a terminal or another electronic device connected to the device for measuring the confidence of the molecular structure prediction model 600 in a wired or wireless communication manner. For example, the transceiver 610 may obtain the first molecular structure image 201, the first molecular structure graph 203 that are determined using the molecular structure prediction model, and the like from another electronic device.

Various types of data, such as programs including applications and files, may be installed and stored in the memory 620. The processor 640 may access and use the data stored in the memory 620, or may store new data in the memory 620. In addition, the memory 620 may store one or more instructions. The processor 640 may execute the one or more instructions stored in the memory.

The processor 640 may control the overall operation of the device for measuring the confidence of the molecular structure prediction model 600, and may include at least one processor, such as a CPU, a GPU, or the like. The processor 640 may control other components included in the device for measuring the confidence of the molecular structure prediction model 600 to perform operations for operating the device for measuring the confidence of the molecular structure prediction model 600. For example, the processor 640 may obtain the first molecular structure image 201, obtain the first molecular structure graph 203 determined using the first molecular structure prediction model 210, perform image rendering on the first molecular structure image 201 based on the first molecular structure graph 203, and determine the confidence of the first molecular structure graph 203 based on the image rendering result 205 and the first molecular structure graph 203.

The database 630 may store various training data for training the learning model. In addition, material information, phase information, simulation result information, and the like may be stored in the database 630, and in various embodiments, output data generated by the learning model may also be stored. Although FIG. 6 shows that the device for measuring the confidence of the molecular structure prediction model 600 includes the database 630, the database 630 may be provided outside the device for measuring the confidence of the molecular structure prediction model 600. In this case, the database 630 may be connected to the device for measuring the confidence of the molecular structure prediction model 600 in a wired or wireless communication manner.

Furthermore, the learning model may be implemented outside the device for measuring the confidence of the molecular structure prediction model 600 (e.g., implemented in a cloud-based manner), or may be included in the device for measuring the confidence of the molecular structure prediction model 600.

One embodiment of the invention may be implemented in the form of a recording medium including computer-executable instructions, such as program modules executed by a computer. A computer-readable medium may be any available medium that can be accessed by the computer, and may include all of volatile and non-volatile media, and removable and non-removable media. In addition, the computer-readable medium may include both a computer storage medium and a communication medium. The computer storage medium may include all of volatile and non-volatile, removable and non-removable media that are implemented in any method or technology for storing information such as computer-readable instructions, data structures, program modules, or other data. The communication medium typically includes computer-readable instructions, data structures, or program modules and includes any information transmission medium.

According to one embodiment of the invention, accuracy information about a result of a prediction model may be provided by providing the confidence of the result of the prediction model together, and furthermore, it may help a user determine which information to store in a database.

The above description of the present disclosure is for illustrative purposes, and those skilled in the art to which the present disclosure pertains will understand that various modifications can be easily made into other specific forms without departing from the technical spirit or essential characteristics of the present invention. Therefore, it should be understood that the above-described embodiments are illustrative and not restrictive in all respects. For example, each component described in a singular form may be implemented separately, and likewise, components described as being implemented separately may also be implemented in a combined form.

The scope of the present disclosure is defined by the appended claims rather than the above detailed description, and all changes or modifications derived from the meaning and scope of the claims and their equivalent concepts should be construed as being included within the scope of the present disclosure.

## Claims

1. A system for measuring the confidence of a molecular structure prediction model, comprising:
a memory storing one or more instructions; and
at least one processor configured to execute the one or more instructions stored in the memory,
wherein:
the at least one processor, by executing the one or more instructions,
obtains a first molecular structure image;
obtains a first molecular structure graph determined using the molecular structure prediction model;
performs image rendering on the first molecular structure image based on the first molecular structure graph; and
determines the confidence of the first molecular structure graph based on the image rendering result and the first molecular structure graph.

2. The system of claim 1, wherein:
the at least one processor
identifies at least one of a first component and a second component based on the first molecular structure graph;
identifies a first portion corresponding to the first component in the first molecular structure image;
identifies a second portion corresponding to the second component in the first molecular structure image; and
performs the image rendering by distinguishing the first portion and the second portion using different markings; and
each of the first component and the second component includes one of a first atom, a second atom, a first bond, and a second bond.

3. The system of claim 1, wherein the molecular structure prediction model includes a first learning model trained to extract a chemical table file graph with a molecular structural formula image as input.

4. The system of claim 1, wherein the at least one processor outputs the confidence using a second learning model with the image rendering result and the first molecular structure graph as input.

5. The system of claim 4, wherein the second learning model includes:
an image backbone model configured to extract a feature of the image rendering result;
a graph backbone model configured to extract a feature of the first molecular structure graph;
a feature concatenation unit configured to concatenate the feature of the image rendering result and the feature of the first molecular structure graph; and
a linear layer model configured to determine the confidence with an output of the feature concatenation unit as input.

6. The system of claim 4, wherein the second learning model is trained to output a first value when the image rendering result matches the first molecular structure graph and to output a second value when the image rendering result does not match the first molecular structure graph.

7. The system of claim 1, wherein the graph of the molecular structure with the confidence equal to or greater than a predetermined level is stored in a database.

8. A method for measuring the confidence of a molecular structure prediction model, performed by at least one processor, comprising:
obtaining a first molecular structure image;
obtaining a first molecular structure graph using the molecular structure prediction model;
performing image rendering on the first molecular structure image based on the first molecular structure graph; and
determining the confidence of the first molecular structure graph based on the image rendering result and the first molecular structure graph.

9. The method of claim 8, wherein:
the performing of the image rendering on the first molecular structure image includes:
identifying at least one of a first component and a second component based on the first molecular structure graph;
identifying a first portion corresponding to the first component in the first molecular structure image;
identifying a second portion corresponding to the second component in the first molecular structure image; and
performing the image rendering by distinguishing the first portion and the second portion using different markings; and
each of the first component and the second component includes one of a first atom, a second atom, a first bond, and a second bond.

10. The method of claim 8, wherein the molecular structure prediction model includes a first learning model trained to extract a chemical table file graph with a molecular structural formula image as input.

11. The method of claim 8, wherein the determining of the confidence of the first molecular structure graph includes outputting the confidence of the first molecular structure graph using a second learning model with the image rendering result and the first molecular structure graph as input.

12. The method of claim 11, wherein the second learning model includes:
an image backbone model configured to extract a feature of the image rendering result;
a graph backbone model configured to extract a feature of the first molecular structure graph;
a feature concatenation unit configured to concatenate the feature of the image rendering result and the feature of the first molecular structure graph; and
a linear layer model configured to determine the confidence with an output of the feature concatenation unit as input.

13. The method of claim 11, wherein the second learning model is trained to output a first value when the image rendering result matches the first molecular structure graph and to output a second value when the image rendering result does not match the first molecular structure graph.

14. The method of claim 8, wherein the graph of the molecular structure with the confidence equal to or greater than a predetermined value is stored in a database.

15. A program stored on a computer-readable recording medium to execute the method of any one of claims 8 to 14 on a computer.
